# EUROPÄISCHE PATENTANMELDUNG

(11) **EP 2 228 010 A1**
(43) Veröffentlichungstag der Anmeldung: **15.09.2010**
(21) Anmeldenummer: 09002412.6
(22) Anmeldetag: 20.02.2009
(51) Int. Cl.: A61B 5/103

(54) **Verfahren zur Ermittlung der Auswirkung von Schuheinlagen auf das Gangbild einer Person beim Laufen**

(71) Anmelder: Schuh + Sport Molitor GmbH, 49084 Osnabrück (DE)
(72) Erfinder: Molitor, Dirk, 49084 Osnabrück (DE)
(74) Vertreter: Quermann, Helmut

(57) **Zusammenfassung**

Die Erfindung betrifft ein Verfahren zur Ermittlung der Auswirkung von Schuheinlagen auf das Gangbild einer Person beim Laufen, insbesondere beim horizontalen Gehen oder Bergabgehen.

Das Verfahren weist folgende Merkmale auf:
a. Eingangserfassung des Bewegungsmusters der Person im Bereich der Kniegelenke beim Laufen ohne Schuheinlagen zu Referenzzeitpunkten beim initialen Kontakt bis zum Ende der Hauptstoßdämpfungsphase des jeweiligen Fußes der Person,
b. Auswertung des erfassten Bewegungsmusters unter dem Aspekt der Änderung des Q-Winkels des jeweiligen Knies zwischen den Referenzzeitpunkten,
c. Herstellung einer individuellen Schuheinlage, deren Gestaltung der Änderung des Q-Winkels des jeweiligen Knies zwischen den Referenzzeitpunkten entgegenwirkt,
d. Kontrollerfassung zu den Referenzzeitpunkten des Bewegungsmusters dieser Person im Bereich deren Kniegelenke beim Laufen mit den hergestellten Schuheinlagen,
e. Auswertung des erfassten Bewegungsmusters bei Verwendung der hergestellten Schuheinlagen unter dem Aspekt der Änderung des Q-Winkels des jeweiligen Knies zwischen den Referenzzeitpunkten.

## Beschreibung

Die Erfindung betrifft ein Verfahren zur Ermittlung der Auswirkung von Schuheinlagen auf das Gangbild einer Person beim Laufen, insbesondere beim horizontalen Gehen oder Bergabgehen.

Aus der DE 44 18 475 A1 ist ein Verfahren und eine Messanordnung zur Analyse des menschlichen Ganges bekannt. Hierbei werden durch Messung der beim Gehen auftretenden kinematischen und kinetischen Wirkungen, aus denen Rückschlüsse auf den Zustand des Stütz- und Bewegungsapparates gezogen werden können, zunächst für die jeweilige Testperson mittels eines Laufbandes ein bestimmtes, reproduzierbares Laufregime eingestellt und eine im Wesentlichen gleichbleibende Laufphase durch Energieverbrauchsmessung ermittelt. Während der konstanten Laufphase wird mit Drucksensoren die von den Füßen ausgehende, dynamische Druckwirkung zusammen mit der durch zwei - dimensionale Winkelmessung festgestellten Lageänderung ausgewählter Körpersegmente zueinander bestimmt sowie eine Muskelpotenzialmessung vorgenommen. Gleichzeitig wird das Laufverhalten der Person überwacht. Die das vorgegebene Laufregime und das gleichmäßige Laufverhalten bestimmenden Daten sowie die Messergebnisse am Bewegungs- und Stützapparat des Menschen werden von einem Rechner online überwacht und gesteuert bzw. online dargestellt.

Ein System zur Betrachtung von Bewegungsabläufen des menschlichen Ganges ist in der DE 20 2007 006 221 U1 beschrieben. Das System weist ein Bewegungsbereich für eine Person, eine Aufzeichnungsvorrichtung, eine Datenverarbeitungsvorrichtung und ein Wiedergabegerät auf. Die Aufzeichnungsvorrichtung ist so positioniert, dass sie die Person, die sich im Bewegungsbereich befindet, von mindestens einer oder zwei, vorzugsweise allen Seiten aufzuzeichnen in der Lage ist. Es werden Aufzeichnungsdaten erstellt, die über die Datenverarbeitungsvorrichtung an das mindestens eine Wiedergabegerät weitergegeben und von diesem für den Bewegenden, als Bewegungsselbstkorrektur und Wahrnehmungshilfe, sichtbar und simultan zu dessen Bewegung ausgegeben werden.

Das bekannte Verfahren bzw. bekannte System stehen in keinem Zusammenhang mit der Analyse des menschlichen Ganges unter dem Aspekt der Ermittlung der Auswirkung von Schuheinlagen auf das Gangbild der Person beim Laufen.

Das Laufen, sei es mit höherer oder geringerer Geschwindigkeit, insbesondere das mit geringerer Geschwindigkeit erfolgende Gehen, ist oftmals mit erheblichen Kniebeschwerden verbunden. Dies gilt besonders für den Fall des Bergabgehens, beispielsweise während Wanderungen, bei denen über einen längeren Zeitraum Bergab gegangen wird und demzufolge durchaus unter erhöhter Belastung durch Gepäck, das getragen wird, die Knie erheblichen Beanspruchungen ausgesetzt sind und demnach Kniebeschwerden auftreten.

Aufgabe der vorliegenden Erfindung ist es, ein Verfahren anzugeben, das es gestattet, die Auswirkung von Schuheinlagen auf das Gangbild einer Person beim Laufen, insbesondere beim horizontalen Gehen oder Bergabgehen zu ermitteln.

Gelöst wird die Aufgabe durch ein Verfahren zur Ermittlung der Auswirkung von Schuheinlagen auf das Gangbild einer Person beim Laufen, insbesondere beim horizontalen Gehen oder Bergabgehen, mit folgenden Merkmalen:
a. Eingangserfassung des Bewegungsmusters der Person im Bereich der Kniegelenke beim Laufen ohne Schuheinlagen zu Referenzzeitpunkten beim initialen Kontakt bis zum Ende der Hauptstoßdämpfungsphase des jeweiligen Fußes der Person,
b. Auswertung des erfassten Bewegungsmusters unter dem Aspekt der Änderung des Q-Winkels des jeweiligen Knies zwischen den Referenzzeitpunkten,
c. Herstellung einer individuellen Schuheinlage, deren Gestaltung der Änderung des Q-Winkels des jeweiligen Knies zwischen den Referenzzeitpunkten entgegenwirkt,
d. Kontrollerfassung zu den Referenzzeitpunkten des Bewegungsmusters dieser Person im Bereich deren Kniegelenke beim Laufen mit den hergestellten Schuheinlagen,
e. Auswertung des erfassten Bewegungsmusters bei Verwendung der hergestellten Schuheinlagen unter dem Aspekt der Änderung des Q-Winkels des jeweiligen Knies zwischen den Referenzzeitpunkten.

Wesentlich ist bei dem erfindungsgemäßen Verfahren, dass eine Eingangserfassung des Bewegungsmusters der Person und eine Auswertung des erfassten Bewegungsmusters der Eingangserfassung erfolgt, um aufgrund der gewonnenen Erkenntnisse individuelle Schuheinlagen für die Person herzustellen. Anschließend werden die Auswirkungen der individuellen Schuheinlagen im Rahmen einer Kontrollerfassung des Bewegungsmusters dieser Person ermittelt. Die dann folgende Auswertung des erfassten Bewegungsmusters der Kontrollerfassung ermöglicht Aussagen im Sinne einer Ergebnisqualitätskontrolle, somit einer positiven Auswirkung der Schuheinlage. Von besonderer Bedeutung ist dabei, dass die Eingangserfassung und die Kontrollerfassung das Bewegungsmuster der Person im Bereich deren Kniegelenken beim Laufen zum Zeitpunkt des initialen Kontakts (IC - Initial Contact) bis zum Zeitpunkt des Endes der Hauptstoßdämpfungsphase (Ende LR - Ende Loading Response) des jeweiligen Fußes erfasst wird. Für diese Phase gilt die Definition von Kirsten Götz-Neumann gemäß Buch "Gehen Verstehen", Thieme Verlag.

Die Eingangserfassung des Bewegungsmusters der Person und/oder die Kontrollerfassung des Bewegungsmusters dieser Person erfolgt insbesondere optisch. Andere Arten der Erfassung sind durchaus denkbar, beispielsweise die Erfassung des Bewegungsmusters mittels Lagesensoren oder aktiven Sendern, ferner durch goniometrische Verfahren oder mittels Gyroskop.

Von besonderer Bedeutung ist bei dem erfindungsgemäßen Verfahren ferner, dass die Erfassung der Bewegungsmuster von Eingangserfassung und Kontrollerfassung unter dem Aspekt der Änderung des Q-Winkels des jeweiligen Knies zwischen diesen Referenzzeitpunkten, somit dem ersten Referenzzeitpunkt des initialen Kontakts und dem zweiten Referenzzeitpunkt des Endes der Hauptstoßdämpfungsphase des jeweiligen Fußes, erfolgt.

So ist der Q-Winkel (Quadrizepssehnenwinkel) definiert bezüglich seines Scheitelpunktes durch die Patellamitte, seines einen Schenkels durch den Femurschenkel des Beins und des anderen Schenkels durch den Patellasehnenschenkel des Beins. Der zwischen diesen beiden Teilwinkeln vorhandene 180°-Winkel wird als Neutrainull definiert und demnach nicht bei der Bewertung berücksichtigt. Zur Bestimmung des Kniewinkels werden an der Person folgende anatomische Punkte markiert: Patellamitte - SIAS (Spina iliaca anterior superior) - Tuberositas.

Das Ende der LR-Phase ist interessant, weil hier bei maximaler Beinlast erfahrungsgemäß die meisten Beschwerden am Knie auftreten.

Zur maximalen Beinlast ist maximale Knieflexion und maximaler Pronationsanschlag im unteren Sprunggelenk (USG) zu erwarten. Diese Momente nehmen Einfluss auf die Kniesteuerung. Demzufolge bedingt ein Einfluss auf Knieflexion und unteres Sprunggelenk (USG) über den Fuß das Knie. In Abhängigkeit von der Form der Einlage zeigt somit das Schienenbein am Tibiakopf Veränderungen.

Aufgrund der insbesondere optischen Erfassung des Bewegungsmusters während der Eingangserfassung und der Kontrollerfassung und der Auswertung der Bewegungsmuster kann analysiert werden, wie weit beim Laufen zu den definierten Referenzzeitpunkten eine Valgisierung des jeweiligen Knies ausgelöst wird. Diese Änderung des Q-Winkels, somit die Bewegung des jeweiligen Knies am Ende LR zum Bezugspunkt IC im Sinne einer Tendenz medial oder lateral, vermittelt eine Information dahingehend, ob das vorherrschende Drehmoment im Bereich des Knies laterale oder mediale Ausrichtung zeigt. Hierbei ist das vorherrschende Drehmoment von vielen Faktoren beeinflussbar. Das externe Drehmoment am Knie ist aufgrund der Körperschwerpunktlage - der Körperschwerpunkt liegt in der Regel zwischen beiden Knien - immer varisch, also lateral. Die Muskulatur steuert gegenläufig und bildet ein internes Drehmoment. Das vorherrschende Drehmoment ist also ein Ergebnis aus externen und internen Anforderungen. Neben der Muskulatur sind die Gelenkbeweglichkeit und die Stellung der Gelenkflächen zueinander weitere wichtige Faktoren, die das vorherrschende Drehmoment, natürlich nicht nur das im Bereich des Kniegelenks, beeinflussen. Welche Artikulation diese Gelenke, am Fuß, an der Hüfte und natürlich das Kniegelenk selbst zulassen, beeinflusst somit auch das vorherrschende Drehmoment am Knie. Unter dem Aspekt der Herstellung der individuellen Schuheinlage ist es wesentlich, einlagentechnisch die Kniesteuerung so zu beeinflussen, dass es zu einer Beschwerdelinderung kommt. Die gewünschte Ausrichtung der Steuerung ist demnach dem zuvor ermittelten vorherrschenden Drehmoment gegenläufig vorzusehen. Dem vorherrschenden Drehmoment ist, wie ausgeführt, der Q-Winkel zugeordnet. Durch Veränderung des Q-Winkels lässt sich das Drehmoment verändern.

Ist beispielsweise das vorherrschende Drehmoment lateral, ist eine valgische Ausrichtung des Q-Winkels zur Beschwerdelinderung notwendig. Ist der Unterschenkel verantwortlich, so muss die Einlage den Patellasehnenwinkel vergrößern.

Ist beispielsweise das vorherrschende Drehmoment medial, ist eine varische Ausrichtung des Q-Winkels zur Beschwerdelinderung notwendig. Ist der Unterschenkel verantwortlich, so muss die Einlage den Patellasehnenwinkel verkleinern.

Gemäß einer bevorzugten Weiterbildung der Erfindung ist vorgesehen, dass die erfassten Bewegungsmuster dahingehend ausgewertet werden, inwieweit die Veränderung der Lage des Patellasehnenschenkels des Q-Winkels die Veränderung des Q-Winkels beeinflusst. Zeigt sich der Femurschenkel für die Vergrößerung oder Verkleinerung des Q-Winkels verantwortlich, sind konsequent physiotherapeutische Maßnahmen zur Beschwerdelinderung hilfreich. Ist der Patellasehnenschenkel des Q-Winkels für die Vergrößerung oder Verkleinerung des Q-Winkels verantwortlich, so sind einlagentechnische Maßnahmen zur Beschwerdelinderung hilfreich. Über den Fuß wird die Unterschenkelausrichtung beeinflusst. Der Tibiakopf verändert so seine Position und der Patellasehnenwinkel vergrößert und verkleinert sich. Hierauf zielt die vorliegende Erfindung ab.

Vorzugsweise werden die erfassten Bewegungsmuster nicht nur bezüglich des Q-Winkels, der Lage des jeweiligen Schenkels des Q-Winkels zur Vertikalen, sondern auch bezüglich des Kniebeugewinkels und der Kniesteuerung zur Einschätzung des vorherrschenden, in das Knie eingeleiteten Drehmoments ausgewertet. Dies insbesondere unter dem Aspekt, dass ein Q-Winkel am Ende der Phase LR von 9° bis 15° als adäquat und ein solcher unter 9° oder über 15° als inadäquat angesehen wird. Die Erfassung des Kniebeugewinkels, somit des Winkels, der die Beugung des Knies von IC bis Ende LR beschreibt, ist deshalb von Bedeutung, weil ab 30° Knieflexion, somit einem Kniebeugewinkel von 30°, die Knieseitenbänder am Knie nicht stabilisieren. Je mehr Knieflexion ein Kniegelenk zur Stoßdämpfungsphase zeigt, umso mehr Q-Winkelausschlag ist zu erwarten. Besonders beim Gehen bergab ist der Kniebeugewinkel von besonderem Interesse, da hier ein verstärktes Flexionsdrehmoment am Kniegelenk vorliegt.

Dieses Parameter ist von Bedeutung bei Kniebeugekontraktionen. Oftmals sind diese Kontrakturen auch schmerzbedingt. Je weniger Flexion ein Kniegelenk zur Stoßdämpfungsphase zeigt (bei unter 10° Flexion spricht die Literatur von Kontrakt), umso weniger Q-Winkelausschlag ist zu erwarten.

Die optische Eingangserfassung/Kontrollerfassung der Bewegungsmuster und deren Auswertung erfolgt vorzugsweise mittels computerunterstützter Video-Laufanalyse.

Das erfindungsgemäße Verfahren findet insbesondere Verwendung zur Ermittlung der Auswirkung von Schuheinlagen auf das Gangbild einer gehenden Person, insbesondere einer Person, die auf einem Laufband geht. Vorzugsweise werden die Bewegungsmuster bei maximal möglicher Gehgeschwindigkeit der Person erfasst.

Grundsätzlich kann die Eingangserfassung des Bewegungsmusters der Person vorgenommen werden, wenn diese barfuß läuft. Vorteilhaft erfolgt aber die Eingangserfassung des Bewegungsmusters beim Laufen in Neutralschuhen. Bei einem Neutralschuh handelt es sich um eine Ummantelung des Fußes, die dem Zweck dient, Einbauelemente der Fußsohle anzufügen, ohne dass weitere Parameter als die eingefügten Elemente die Fußsohle beeinflussen. Ein Neutralschuh ist in aller Regel ein Gummischuh aus gleichmäßiger Materialstärke mit zwei Klettverschlüssen, um ihn am Fußrücken zu schließen. Er hat in aller Regel keinen Absatz, ist flexibel und in mehreren Größen verfügbar. Der Neutralschuh ist notwendig, um die Wirkungsweise von Einlagen, ohne Beeinflussung anderer Faktoren, zu testen. Durch Verwendung von Neutralschuhen wird verhindert, dass die Laufunterlage, beispielsweise ein profiliertes Laufband, nachteilig unmittelbar auf die Fußsohle bzw. den Fuß einwirkt und somit eine Änderung des Bewegungsmusters der Person bedingt. Zweckmäßig erfolgt auch die Kontrollerfassung des Bewegungsmusters bei Verwendung derselben Neutralschuhe, in die dann die hergestellten Einlagen eingelegt sind.

Bei der Schuheinlage handelt es sich vorzugsweise um eine solche, die als Basis-Einlage eine gegossene Schaleneinlage aufweist oder es sind mit der Basis-Einlage Module verbunden, insbesondere verklebt.

Gemäß einer besonderen Weiterbildung der Erfindung ist vorgesehen, dass in Korrelation zu der Erfassung der Eingangs- und Kontrollbewegungsmuster Schmerzbilder der Person erfasst werden. Insbesondere wird vor der optischen Eingangserfassung des Bewegungsmusters der Person eine Anamnese und Lokalisation und Markierung der Kniebeschwerden durch den Patienten durchgeführt. Insbesondere wird dies durch den Patient mit einem Stift an den eigenen Knien markiert. Ein Foto der Markierung wird vorzugsweise im Rahmen der computerunterstützten Video-Laufanalyse dokumentiert. Der Schmerzfaktor wird mittels der visuell-analogen Schmerzskala (VAS) von 1 - 10 bestimmt und dokumentiert. Vor der optischen Kontrollerfassung des Bewegungsmusters nach mehrwöchiger Nutzung der Schuheinlagen, insbesondere nach etwa sechswöchiger Nutzung der Schuheinlagen, wird erneut mit Hilfe der visuellen analogen Schmerzskala der Schmerzfaktor abgefragt.

Ist die Auswirkung der Schuheinlagen auf das Gangbild der Person noch nicht optimal, erfolgt eine Nachbearbeitung bzw. Veränderung der Schuheinlagen, Es schließen sich demnach an die Verfahrensabfolge gemäß der Verfahrensschritte d. und e. zumindest ein weiteres Mal die Verfahrensabfolge gemäß der Verfahrensschritte c. und d. und e. an.

Die Erfindung beschreibt somit ein Analyseverfahren zur Herstellung und zur Ergebniskontrolle der Wirkung von Schuheinlagen bei Kniebeschwerden beim Laufen, insbesondere beim horizontalen Gehen oder Bergabgehen.

Nachfolgend sind für eine bevorzugte Ausführungsform des erfindungsgemäßen Verfahrens die einzelnen Detailschritte beschrieben, ohne hierauf beschränkt zu sein.

### Schritt 1:

### Darstellung/Beschreibung der Kniebeschwerden

- Anamnese und Lokalisation und Markierung der Kniebeschwerden durch den Patienten (Patient markiert mit Stift am eigenen Knie) - Ein Foto der Markierung wird dokumentiert.
- Schmerzfaktor wird mittels der visuell-analogen Schmerzskala (VAS) von 1-10 bestimmt und dokumentiert.

### Schritt 2:

### Videoanalyse

Mittels Videoanalyse wird die Kniebewegung auf dem Laufband unter maximal möglicher Gehgeschwindigkeit untersucht. Der Patient benutzt dabei Neutralschuhe ohne Einlagen. Die Kniesteuerung wird zum Zeitpunkt des initialen Kontakts (IC) untersucht, bis zum Zeitpunkt des Endes der Hauptstoßdämpfungsphase (Ende LR). (Definition Götz-Neumann).

Es gehört zum besonderen Merkmal der Erfindung, diese zwei Phasen miteinander zu vergleichen.

Der Zeitpunkt des initialen Kontakts ist interessant, weil das Bein komplett unbelastet ist. Der Zeitpunkt des Endes der LR-Phase ist interessant, weil hier bei maximaler Beinlast die meisten Beschwerden am Knie auftreten. Bei maximaler Beinlast ist die maximale Knieflexion und der maximale Pronationsanschlag im unteren Sprunggelenk zu erwarten.

Zur Bestimmung des Kniewinkels (Q-Winkels) werden folgende anatomische Punkte markiert: Patellamitte - SIAS - Tuberositas. Der Q-Winkel ergibt sich aus der Summe von Femurwinkel F und Patellasehnenwinkel P. Hierbei bleibt der als Neutralnull definierte Winkelanteil von 180° beim Kniewinkel aus Vereinfachungsgründen außer Acht (Kniewinkel = 180° + F + P), so dass, aufgrund dieser Neutralnull-Definition, Q = F + P ist.

### 1. Parameter

### Beziehung Kniewinkel (Q-Winkel) zum Verhältnis der Schenkel zueinander

- Ist der Q-Winkel am Ende-LR adäquat (9°-15°) oder inadäquat (unter 9°, über 15°)? Ja-nein
- Q-Winkel: Vergrößert sich der Winkel am Ende LR zum IC? Ja-nein
- Ist der Patellasehnenwinkei für die Vergrößerung oder Verkleinerung des Q-Winkels verantwortlich?
   Ist der Femurwinkel für die Vergrößerung oder Verkleinerung des Q-Winkels verantwortlich?

Dieses Verfahren dient der Analyse, inwieweit eine Valgisierung des Knies ausgelöst wird und welcher Schenkel verantwortlich ist.

Zeigt sich der Femurwinkel verantwortlich, sind konsequent physiotherapeutische Maßnahmen zu Beschwerdelinderung hilfreich.

Zeigt sich der Patellasehnenwinkel verantwortlich, so sind einlagentechnische Maßnahmen zur Beschwerdelinderung hilfreich. Über den Fuß wird die Unterschenkelausrichtung beeinflusst. Der Tibiakopf verändert so seine Position, und der Patellasehnenwinkel vergrößert oder verkleinert sich.

Dadurch lassen sich eine Vielzahl von klassischen Beschwerdebildern am Knie behandeln:
Patellofemurales Schmerzsyndrom, Tractusiliotibiales Schmerzsyndrom, Pesanserinus Schmerzsyndrom, mediale und laterale Meniskenbeschwerden, Kniegelenkarthrose, usw.. Die Datenbank der Beschwerdebilder wird entsprechend weiteren Therapieerfolgen vervollständigt.

Es gehört zum Merkmal der Erfindung, dass Schmerzbilder in Korrelation zu einer Bewegungsmessung erfasst werden. Ein Merkmal der Erfindung ist es, ein schmerzproduzierbares Bewegungsmuster zu erfassen, um dann ein schmerzreduzierendes Bewegungsmuster zu erzeugen. Eine Bewegung wird erzeugt, die aus dem Beschwerdemuster führt.

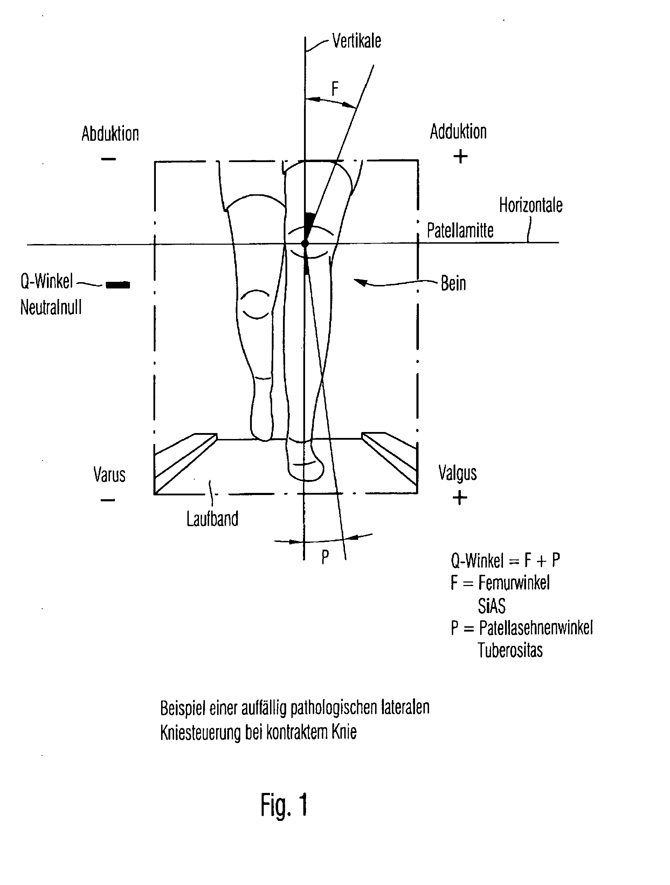

Figur 1 zeigt im Zusammenhang mit einem auf einem Laufband laufenden Patienten (in diesem Fall barfußlaufenden Patienten) bezüglich dessen einen Beines den Q-Winkel und seine Winkelanteile F und P betreffend Femurschenkel und Patellasehnenschenkel, bezogen auf eine vertikale Linie, bei Neutralnull-Definition.

### 2. Parameter

### Erfassung des Kniebeugewinkels

- Wie viel Grad beugt sich das Knie von IC bis Ende LR?

Es ist bekannt, dass ab 30° Knieflexion die Knieseitenbänder das Knie nicht stabilisieren. Je mehr Knieflexion ein Kniegelenk zur Stoßdämpfungsphase zeigt, um mehr Q-Winkelausschlag ist somit zu erwarten. Besonders für das Gehen bergab ist der Kniebeugewinkel von besonderem Interesse, da hier ein verstärktes Flexionsdrehmoment am Kniegelenk vorliegt.

Dieses Parameter wird wichtig bei Kniebeugekontraktionen. Oftmals sind diese Kontrakturen auch schmerzbedingt. Je weniger Flexion ein Kniegelenk zur Stoßdämpfungsphase zeigt (bei unter 10° Flexion spricht die Literatur von kontrakt), um so weniger Q-Winkelausschlag ist zu erwarten.

### 3. Parameter

### Erfassung der Kniesteuerung zur Einschätzung des vorherrschenden Drehmomentes am Kniegelenk

Hier wird mittels Videoanalyse nur das Bewegungsmuster der markierten Tubeositas erfasst. Die Ausrichtung wird in mm gemessen.
- Wohin bewegt sich das Knie am Ende LR zum Bezugspunkt IC? /medial/-lateral /Tendenz:

Es gilt festzustellen, ob das vorherrschende Drehmoment laterale oder mediale Ausrichtung zeigt. Das vorherrschende Drehmoment ist von vielen Faktoren beeinflussbar. Das externe Drehmoment am Knie ist aufgrund der Körperschwerpunktlage - der Körperschwerpunkt liegt in der Regel zwischen beiden Knie - immer varisch also lateral. Die Muskulatur steuert gegenläufig und bildet ein internes Drehmoment. Das vorherrschende Drehmoment ist also ein Ergebnis aus externen und internen Anforderungen. Neben der Muskulatur sind die Gelenkbeweglichkeit und die Stellung der Gelenkflächen zueinander weitere wichtige Faktoren, die das vorherrschende Drehmoment beeinflussen. Hier natürlich nicht nur die des Kniegelenkes. Welche Artikulation diese Gelenke, am Fuß, an der Hüfte und natürlich das Kniegelenk selbst, zulassen, beeinflusst somit auch das vorherrschende Drehmoment am Knie.

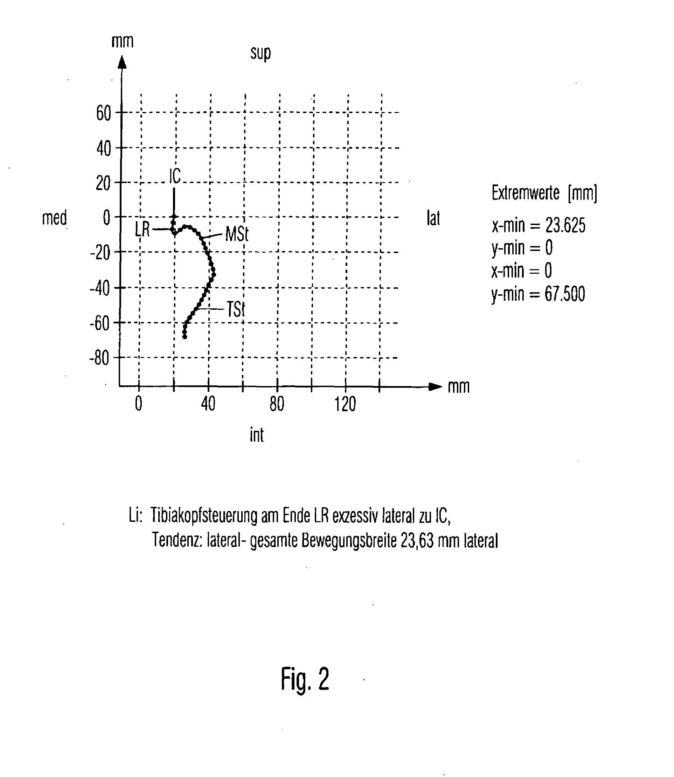

Figur 2 veranschaulicht das Beispiel einer auffällig pathologischen lateralen Kniesteuerung bei kontraktem Knie.

Wie der Figur 2 zu entnehmen ist, wird nicht nur der Q-Winkel zum Zeitpunkt IC mit dem Q-Winkel zum Zeitpunkt Ende LR verglichen, sondern es wird auch das vorherrschende Drehmoment am Kniegelenk berücksichtigt, ermittelt in der Frontalansicht durch das Verlaufsmuster des Tibiakopfes von IC bis vier Bilder in die mittlere Stützphase (MSt) hinein (Parameter 3). Es ist so eine Aussage dahingehend zu treffen, wohin sich das Knie am Ende LR zum Bezugspunkt IC hin bewegt - medial, lateral, Tendenz medial oder lateral. Es wird so an einem wichtigen Punkt - am Ende LR - aus einer Bewegungskurve eine Probe entnommen. Diese "Probe" zeigt den Q-Winkel am Ende LR. Der Q-Winkel soll durch die Einlage beeinflusst werden. Die Intervention zielt auf die Stellung des Winkels zu einem definierten Zeitpunkt, um dann durch einlagentechnische Maßnahmen das Drehmoment des Knies positiv, insbesondere im Sinne einer Schmerzlinderung, zu beeinflussen.

### Schritt. 3:

### Zielbestimmung

Es gilt einlagentechnisch die Kniesteuerung zu beeinflussen, so dass es zur Beschwerdelinderung kommt. Die gewünschte Ausrichtung der Steuerung ist dem zuvor ermittelten vorherrschenden Drehmoment gegenläufig. Dem vorherrschenden Drehmoment ist ein Q-Winkel zugeordnet. Durch Veränderung des Q-Winkel wird das Drehmoment verändert:
- Ist das vorherrschende Drehmoment lateral, ist eine valgische Ausrichtung des Q-Winkels zur Beschwerdelinderung notwendig. Ist der Unterschenkel verantwortlich, so muss die Einlage den Patellasehnenwinkel vergrößern.
- Ist das vorherrschende Drehmoment medial, ist eine varische Ausrichtung des Q-Winkels zur Beschwerdelinderung notwendig. Ist der Unterschenkel verantwortlich, so muss die Einlage den Patellasehnenwinkel verkleinern.

### Schritt 4:

### Erfassung der Daten der Eingangsmessung

Der Computer errechnet den Q-Winkel, den Femurwinkel F, den Patellasehnenwinkel P und die Differenzen dieser Winkel - Ende LR-IC. Die Differenzen Femurwinkel und Patellasehnenwinkel Ende LR-IC zeigen am Winkelwert, welcher Schenkel für die Q-Winkeländerung hauptverantwortlich ist.

### LR-IC Femurwinkel:

Negativer Wert - Richtung Abduktion - verkleinert den Q-Winkel
Positiver Wert - Richtung Adduktion - vergrößert den Q-Winkel

### LR-IC Patellasehnenwinkel:

Negativer Wert - Richtung varus - verkleinert den Q-Winkel
Positiver Wert - Richtung valgus - vergrößert den Q-Winkel
   (zum Verständnis siehe Graphik gemäß Figur 1)

Tabellarisch werden die Ergebnisse erfasst, insofern wird auf das unmittelbar folgende Tabellenwerk verwiesen. Es empfiehlt sich, mit Durchschnittswerten aus 3 Phasen zu arbeiten.

| VAS: | | | | Werte | | Zielbestimmung |
|---|---|---|---|---|---|---|
| | | links | Kniebeugewinkel | | | |
| | | | Q-Winkel | #DIV/0! | | |
| | | | Femurwinkel F | #DIV/0! | | |
| | IC | | Patellasehnenwinkel P | #DIV/0! | | |
| | | rechts | Kniebeugewinkel | | | |
| | | | Q-Winkel | #DIV/0! | | |
| | | | Femurwinkel F | #DIV/0! | | |
| Eingangsmessung Pre | | | Patellasehnenwinkel P | #DIV/0! | LR-IC | |
| | | links | Kniebeugewinkel | | | |
| | | | Q-Winkel | #DIV/0! | #DIV/0! | |
| | | | Femurwinkel F | #DIV/0! | #DIV/0! | |
| | LR | | Patellasehnenwinkel P | #DIV/0! | #DIV/0! | |
| | | rechts | Kniebeugewinkel | | | |
| | | | Q-Winkel | #DIV/0! | #DIV/0! | |
| | | | Femurwinkel F | #DIV/0! | #DIV/0! | |
| | | | Patellasehnenwinkel P | #DIV/0! | #DIV/0! | |
| Kniesteuerung | | links | Tendenz: | medial | lateral | |
| | | rechts | Tendenz: | medial | lateral | |

### Schritt 5:

### Konstruktion der Basis-Einlage

Die Grundkonstruktion der Einlage basiert auf elastischem Poliadditionsschaum. Die Einlage ist sehr flexibel gestaltet und bietet nicht starre Eigenschaften. Gewählt wird ein relativ fester Poliadditionsschaum. Er weist auch nach Dauereinsatz keine signifikaten Materialveränderung auf. Die Basis-Einlage ist eine Schaleneinlage, die den Rückfuß bis zu den Mittelfußköpfchen umfasst. Sie wird nach dem teilbelasteten Abdruck des Fußes gegossen. Das Abdruckverfahren wird mittels eines Trittschaums vollzogen, in den der Fuß eingedrückt wird. Die Tibiastellung beim Abdruckverfahren ist in neutral Null. Etwaige Korrekturen am Fuß bleiben bei der Grundkonstruktion unberücksichtigt. Bei der Abdruckabnahme wird lediglich darauf geachtet, dass die Tibia achsengerecht geführt wird und, dass der Ansatz der Plantaraponeurose ausmodelliert ist, weil sich dieser Sehnenansatz als druckempfindlich erweist.

Zur exakten Anpassung der Einlage wird in einem Schleifverfahren die Basis-Einlage durch den Orthopädieschuhmachermeister modifiziert. Die Einlage wird so eingeschliffen, dass die Stellung des Fußes auf der Einlage identisch ist, wie die teilbelastete Stellung des Fußes im Trittschaum. Die Materialstärke im Vorfuß wie Rückfuß wird gleichmäßig, bis zur idealen Passform der Einlage zum Schuhwerk, reduziert.

Da das Abdruckverfahren teilbelastet und mit neutraler Stellung der Tibia entsteht, unterliegt der Fuß keiner belastungsbedingten Deformität. Es ist daher möglich, dass die gewünschte Ausrichtung des Patellasehnenwinkels schon mit der Basis-Einlage erreicht wird.

### Schritt 6:

### Kontrolle 6 Wochen nach Auslieferung der Basis-Einlage

- Abfrage des Schmerzfaktors mit Hilfe der visuellen analogen Schmerzskala
- Nach Markierung erneute Erfassung der Kniewinkelverhältnisse mit Einlage
- Soll-Ist-Werteüberprüfung der Messwerte

Es gehört zum Merkmal der Erfindung, dass die Wirkung der Einlage messbar wird. Mit dem Verfahren können Bewegungsmessungen bei einer Vielzahl von Beschwerdebilder am Knie zur Ergebnisqualitätskontrolle eingesetzt werden.

Die Wirkung der Einlage wird im Neutralschuh auf dem Laufband bei identischen Test- und Messabläufen kontrolliert. Dann wird die relative Schenkelausrichtung errechnet. Da es sich nicht um absolute, sondern relative Werte handelt, die verglichen werden, führt dies zur kompletten Ausschaltung von etwaigen Markierungsfehlern.

### Das Messen mit relativen Werten ist ein besonderes Merkmal der Erfindung

Tibiakopfausrichtung Pre = Patellasehnenwinkel LR - Patellasehnenwinkel IC Tibiakopfausrichtung Post = Patellasehnenwinkel LR - Patellasehnenwinkel IC

Die Differenz zwischen Pre und Post misst in Grad die Veränderung, die durch die Einlagenversorgung entstanden ist. Sie dokumentiert gleichzeitig, ob die gewünschte Ausrichtung des Tibiaschenkels erfolgt ist.

| VAS: | | | | Werte | |
|---|---|---|---|---|---|
| | | | Kniebeugewinkel | | |
| | | links | O-Winkel | #DIV/0! | |
| | | | Femurwinkel F | #DIV/0! | |
| | IC | | Patellasehnenwinkel P | #DIV/0! | |
| | | | Kniebeugewinkel | | |
| | | rechts | Q-Winkel | #DIV/0! | |
| Kontrollmessung Post | | | Femurwinkel F | #DIV/0! | |
| | | | Patellasehnenwinkel P | #DIV/0! | LR-IC |
| | | | Kniebeugewinkel | | |
| | | links | O-Winkel | #DIV/0! | #DIV/0! |
| | | | Femurwinkel F | #DIV/0! | #DIV/0! |
| | LR | | Patellasehnenwinkel P | #DIV/0! | #DIV/0! |
| | | | Kniebeugewinkel | | |
| | | rechts | Q-Winkel | #DIV/0! | #DIV/0! |
| | | | Femurwinkel F | #DIV/0! | #DIV/0! |
| | | | Patellasehnenwinkel P | #DIV/0! | #DIV/0! |

| VAS Pre: | | | | | | |
|---|---|---|---|---|---|---|
| VAS Post: | | | | | | |
| | | links | Kniebeugewinkel | | | |
| | | | Q-Winkel | | | |
| | | | Femurwinkel F | | | |
| Vergleich Eingangsmessung Kontrollmessung Veränderung Post - Pre | IC | | Patellasehnenwinkel P | | | |
| | | rechts | Kniebeugewinkel | | | |
| | | | Q-Winkel | | | |
| | | | Femurwinkel F | | | |
| | | | Patellasehnenwinkel P | Pre: LR-IC | Post: LR-IC | Veränderung |
| | | links | Kniebeugewinkel | | | |
| | | | Q-Winkel | #DIV/0! | #DIV/0! | #DIV/0! |
| | | | Femurwinkel F | #DIV/0! | #DIV/0! | #DIV/0! |
| | LR | | Patellasehnenwinkel P | #DIV/0! | #DIV/0! | #DIV/0! |
| | | rechts | Kniebeugewinkel | | | |
| | | | Q-Winkel | #DIV/0! | #DIV/0! | #DIV/0! |
| | | | Femurwinkel F | #DIV/0! | #DIV/0! | #DIV/0! |
| | | | Patellasehnenwinkel P | #DIV/0! | #DIV/0! | #DIV/0! |

### Schritt 7:

### Modulverfahren

Bei unzureichender Beschwerdeentlastung tritt, zu intensiveren Beeinflussung der Tibiakopfausrichtung, das Modulverfahren in Kraft.
- Der Patellasehnenwinkel P des Q-Winkels wird handwerklich unter modularer Bautechnik erneut beeinflusst. Die Module werden auf die Basis-Einlage geklebt.

Die Module:
1. Modul bei Kniesteuerung Ende LR lateral zu IC
   Kniekeil 3 mm lateral
2. Modul bei Fersenpolster lateral flach und Kniesteuerung in Ende LR lateral zu IC
   Fersenbeinpronation 2 mm
3. Modul bei M. peroneus stark und Fersen valgus exzessiv, **Patellasehnenschenkel valgus von IC zu LR**
   Valgus keil - Fersenerhöhung - bei Neubau medial fest
4. Modul bei **Vorfußkontakt** und Kniesteuerung medial oder **Patellasehnenschenkel valgus (US-X)**
   Stimulation Mfk 1
5. Modul bei Overcrossing
   nur nach Abdruck - Therapeutische Maßnahmen empfehlenswert

### Schritt 8:

### Nach weiteren 6 Wochen erneute Kontrolle

- Verfahrensweise wie unter Schritt 6

### Schritt 9:

### Fertigung eines endgültigen Produktes

Bei Zufriedenstellung wird der entwickelte Prototyp der jeweiligen Einlagen aus einem Guss gefertigt.

Es gehört zum besonderen Merkmal der Erfindung, dass jeder endgültigen Versorgung ein in Korrelation zur Bewegungsmessung und zur Schmerzentlastung individuell entwickelter Einlagenprototyp voraus geht.

## Patentansprüche

1. Verfahren zur Ermittlung der Auswirkung von Schuheinlagen auf das Gangbild einer Person beim Laufen, insbesondere beim horizontalen Gehen oder Bergabgehen, mit folgenden Merkmalen:
a. Eingangserfassung des Bewegungsmusters der Person im Bereich der Kniegelenke beim Laufen ohne Schuheinlagen zu Referenzzeitpunkten beim initialen Kontakt bis zum Ende der Hauptstoßdämpfungsphase des jeweiligen Fußes der Person,
b. Auswertung des erfassten Bewegungsmusters unter dem Aspekt der Änderung des Q-Winkels des jeweiligen Knies zwischen den Referenzzeitpunkten,
c. Herstellung einer individuellen Schuheinlage, deren Gestaltung der Änderung des Q-Winkeis des jeweiligen Knies zwischen den Referenzzeitpunkten entgegenwirkt,
d. Kontrollerfassung zu den Referenzzeitpunkten des Bewegungsmusters dieser Person im Bereich deren Kniegelenke beim Laufen mit den hergestellten Schuheinlagen,
e. Auswertung des erfassten Bewegungsmusters bei Verwendung der hergestellten Schuheinlagen unter dem Aspekt der Änderung des Q-Winkels des jeweiligen Knies zwischen den Referenzzeitpunkten.

2. Verfahren nach Anspruch 1, wobei sich an die Verfahrensabfolge gemäß der Verfahrensschritte d. und e. ein oder mehrmals die Verfahrensabfolge gemäß der Verfahrensschritte c. und d. und e. anschließt.

3. Verfahren nach Anspruch 1 oder 2, wobei die erfassten Eingangswerte mit den erfassten Kontrollwerten verglichen werden.

4. Verfahren nach einem der Ansprüche 1 bis 3, wobei die Eingangserfassung des Bewegungsmusters beim Laufen in Neutralschuhen erfolgt und/oder die Kontrollerfassung des Bewegungsmusters beim Laufen in den mit den hergestellten Einlagen versehenen Neutralschuhen erfolgt.

5. Verfahren nach einem der Ansprüche 1 bis 4, wobei eine Schuheinlage, die den Rückfuß bis zu den Mittelfußköpfchen des Fußes umfasst, hergestellt wird.

6. Verfahren nach einem der Ansprüche 1 bis 5, wobei bei der Schuheinlage die Plantaraponeurose ausmodeliert ist.

7. Verfahren nach einem der Ansprüche 1 bis 6, wobei die Schuheinlage als Basis-Einlage eine gegossene Schaleneinlage aufweist oder mit einer Basis-Einlage der Schuheinlage Module verbunden werden, insbesondere verklebt werden.

8. Verfahren nach einem der Ansprüche 1 bis 7, wobei die Eingangserfassung des Bewegungsmusters und/oder die Kontrollerfassung des Bewegungsmusters optisch erfolgt.

9. Verfahren nach einem der Ansprüche 1 bis 8, wobei mittels computerunterstützter Video-Laufanalyse die Bewegungsmuster erfasst und ausgewertet werden.

10. Verfahren nach einem der Ansprüche 1 bis 9, wobei die Person geht und die Bewegungsmuster bei maximal möglicher Gehgeschwindigkeit der Person erfasst werden, insbesondere die Person auf einem Laufband geht.

11. Verfahren nach einem der Ansprüche 1 bis 10, wobei die erfassten Bewegungsmuster bezüglich des Q-Winkels, der Lage des jeweiligen Schenkels des Q-Winkels zur Vertikalen, des Kniebeugewinkels und der Kniesteuerung zur Einschätzung eines vorherrschenden, in das Knie eingeleiteten Drehmoments ausgewertet werden.

12. Verfahren nach einem der Ansprüche 1 bis 11, wobei die erfassten Bewegungsmuster dahingehend ausgewertet werden, inwieweit die Veränderung der Lage des Patellasehnenwinkels P und/oder des Femurwinkels F des Q-Winkels die Veränderung des Q-Winkels beeinflusst.

13. Verfahren nach einem der Ansprüche 1 bis 12, wobei die Erfassung der Bewegungsmuster und deren Auswertung den Kniebeugewinkel zwischen den Referenzzeitpunkten umfasst.

14. Verfahren nach einem der Ansprüche 1 bis 13, wobei beim Auswerten der Bewegungsmuster mittels Computer dieser den Q-Winkel, den Femurwinkel F, den Patellasehnenwinkel P und die Differenzen von Femurwinkel F und Patellasehenwinkel P zu den Referenzzeitpunkten errechnet.

15. Verfahren nach einem der Ansprüche 1 bis 14, wobei die Kontrollerfassung des Bewegungsmusters nach mehrwöchiger Nutzung der Schuheinlagen, insbesondere nach etwa sechswöchiger Nutzung der Schuheinlagen erfolgt.

16. Verfahren nach einem der Ansprüche 1 bis 15, wobei in Korrelation zu der Erfassung der Eingangs- und Kontrollbewegungsmuster Schmerzbilder der Person erfasst werden.

## Geänderte Patentansprüche

### Geänderte Patentansprüche gemäss Regel 137(2) EPÜ.

**1.** Verfahren zur Ermittlung der Auswirkung von Schuheinlagen auf das Gangbild einer Person beim Laufen, insbesondere beim horizontalen Gehen oder Bergabgehen, mit folgenden Merkmalen:
a. Eingangserfassung des Bewegungsmusters der Person im Bereich der Kniegelenke beim Laufen ohne Schuheinlage zu Referenzzeitpunkten beim initialen Kontakt bis zum Ende der Hauptstoßdämpfungsphase des jeweiligen Fußes der Person,
b. Auswertung des erfassten Bewegungsmusters unter dem Aspekt der Änderung des Q-Winkels des jeweiligen Knies zwischen den Referenzzeitpunkten,
c. Herstellung einer individuellen Schuheinlage, deren Gestaltung der Änderung des Q-Winkels des jeweiligen Knies zwischen den Referenzzeitpunkten entgegenwirkt,
d. Kontrollerfassung zu den Referenzzeitpunkten des Bewegungsmusters dieser Person im Bereich deren Kniegelenke beim Laufen mit den hergestellten Schuheinlagen,
e. Auswertung des erfassten Bewegungsmusters bei Verwendung der her gestellten Schuheinlagen unter dem Aspekt der Änderung des Q-Winkels des jeweiligen Knies zwischen den Referenzzeitpunkten.

**2.** Verfahren nach Anspruch 1, wobei die erfassten Eingangswerte mit den erfassten Kontrollwerten verglichen werden.

**3.** Verfahren nach Anspruch 1 oder 2, wobei die Eingangserfassung des Bewegungsmusters beim Laufen in Neutralschuhen erfolgt und/oder die Kontrollerfassung des Bewegungsmusters beim Laufen in den mit den hergestellten Einlagen versehenen Neutralschuhen erfolgt.

**4.** Verfahren nach einem der Ansprüche 1 bis 3, wobei eine Schuheinlage, die den Rückfuß bis zu den Mittelfußköpfchen des Fußes umfasst, hergestellt wird.

**5.** Verfahren nach einem der Ansprüche 1 bis 4, wobei bei der Schuheinlage die Plantaraponeurose ausmodeliert ist.

**6.** Verfahren nach einem der Ansprüche 1 bis 5, wobei die Schuheinlage als Basis-Einlage eine gegossene Schaleneinlage aufweist oder mit einer Basis-Einlage der Schuheinlage Module verbunden werden, insbesondere verklebt werden.

**7.** Verfahren nach einem der Ansprüche 1 bis 6, wobei die Eingangserfassung des Bewegungsmusters und/oder die Kontrollerfassung des Bewegungsmusters optisch erfolgt.

**8.** Verfahren nach einem der Ansprüche 1 bis 7, wobei mittels computerunterstützter Video-Laufanalyse die Bewegungsmuster erfasst und ausgewertet werden.

**9.** Verfahren nach einem der Ansprüche 1 bis 8, wobei die Person geht und die Bewegungsmuster bei maximal möglicher Gehgeschwindigkeit der Person erfasst werden, insbesondere die Person auf einem Laufband geht.

**10.** Verfahren nach einem der Ansprüche 1 bis 9, wobei die erfassten Bewegungsmuster bezüglich des Q-Winkels, der Lage des jeweiligen Schenkels des Q-Winkels zur Vertikalen, des Kniebeugewinkels und der Kniesteuerung zur Einschätzung eines vorherrschenden, in das Knie eingeleiteten Drehmoments ausgewertet werden.

**11.** Verfahren nach einem der Ansprüche 1 bis 10, wobei die erfassten Bewegungsmuster dahingehend ausgewertet werden, inwieweit die Veränderung der Lage des Patellasehnenwinkels P und/oder des Femurwinkels F des Q-Winkels die Veränderung des Q-Winkels beeinflusst.

**12.** Verfahren nach einem der Ansprüche 1 bis 11, wobei die Erfassung der Bewegungsmuster und deren Auswertung den Kniebeugewinkel zwischen den Referenzzeitpunkten umfasst.

**13.** Verfahren nach einem der Ansprüche 1 bis 12, wobei beim Auswerten der Bewegungsmuster mittels Computer dieser den Q-Winkel, den Femurwinkel F, den Patellasehnenwinkel P und die Differenzen von Femurwinkel F und Patellasehenwinkel P zu den Referenzzeitpunkten errechnet.
